# DEMANDE DE BREVET EUROPEEN

(11) **EP 0 809 988 A1**
(43) Date de publication de la demande: **03.12.1997**
(21) Numéro de dépôt: 97420078.4
(22) Date de dépôt: 27.05.1997
(51) Int. Cl.: A61F 5/01

(54) **Orthèse de poignet universelle**

(30) Priorité: 31.05.1996 FR 9606963
(71) Demandeur: RICHARD FRERES S.A., 42530 Saint Genest Lerpt (FR)
(72) Inventeur: Richard, Dominique, 42160 Bonson (FR)
(74) Mandataire: Perrier, Jean-Pierre

(57) **Abrégé**

Dans cette orthèse ambidextre et dorsale ou palmaire, les poches (12a, 12b), sont réalisées dans la partie centrale de l'enveloppe (2), apte à venir sur le dos de la main ou sous la main, sont munies d'extrémités proximales, fermées et parallèles, et d'extrémités distales ouvertes, tandis que, de première part, les éclisses (3a, 3b) sont munies chacune d'une partie distale coudée (15a, 15b) apte à épouser le dos ou l'intérieur de la main, sont rigides, amovibles et retournables dans leur poche, et ont des longueurs différentes de manière que, lorsqu'elles sont engagées à fond dans leurs poches respectives, la droite S joignant leurs extrémités distales soit sensiblement parallèle à l'axe de répartition des articulations métacarpo-phalangiennes, que de deuxième part, l'enveloppe (2) est prolongée au-delà de la partie ouverte des poches (12a, 12b) par un rabat de fermeture (13) muni de moyens d'accrochage sur les poches et d'une charnière souple (22) apte, par appui sur l'extrémité distale des éclisses (3a, 3b), à se plier sensiblement parallèlement à l'axe (S) de répartition des articulations métacarpo-phalangiennes, à l'aplomb duquel cette charnière est disposée, et que, de troisième part, les sangles de maintien sont indépendantes de l'enveloppe et comprennent une sangle distale (4) coopérant avec le pouce et deux sangles transversales, respectivement, intermédiaire (5) et proximale (6).

## Description

L'invention est relative à une orthèse de poignet universelle, c'est à dire ambidextre et dorsale ou palmaire, destinée à assurer l'immobilisation du poignet en laissant une liberté de mouvement aux seules phalanges.

Elle concerne plus particulièrement les orthèses composées d'une enveloppe souple s'étendant longitudinalement des articulations métacarpo-phalangiennes jusqu'en deçà du poignet et comportant plusieurs poches longitudinales, parallèles et juxtaposées pour des éclisses.

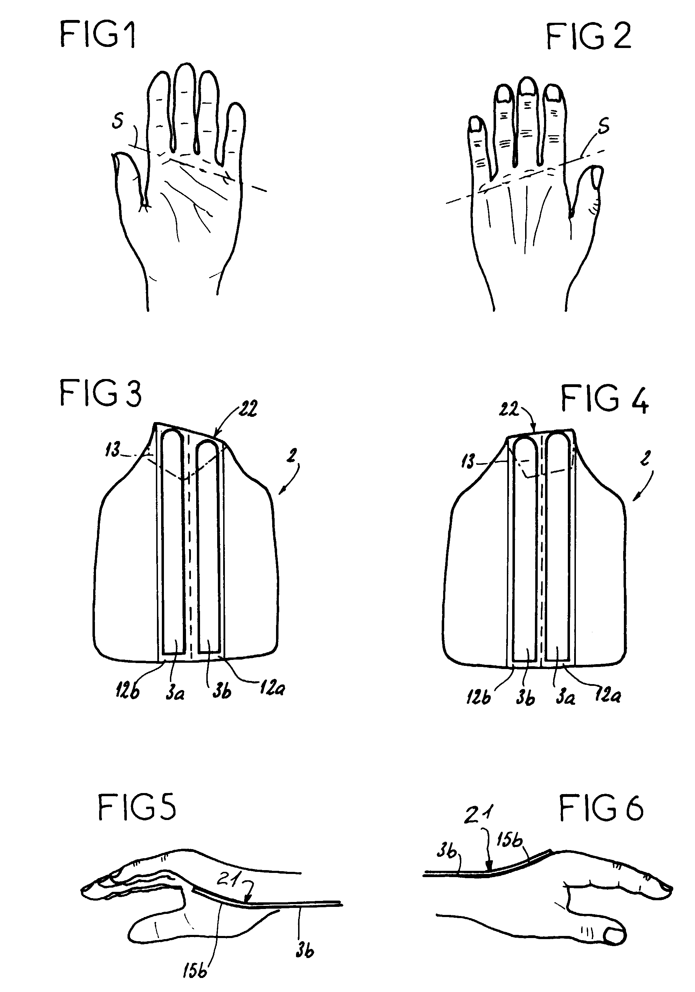

Il est rappelé que la longueur des métacarpes est décroissante de dehors en dedans, comme montré par les traits mixtes aux figures 1 et 2 annexées représentant, respectivement, une main gauche vue de dessous, c'est-à-dire en structure palmaire, et une main gauche vue de dessus, c'est-à-dire en situation dorsale. En raison de cela, toute orthèse destinée à immobiliser le poignet en laissant une liberté de mouvements aux phalanges des doigts, doit prendre en compte cette particularité anatomique.

En pratique, les orthèses existantes sont difficilement ajustables, à moins de multiplier le nombre de modèles en fonction de la diversité des tailles de main, de l'application sur une main gauche ou droite et de l'utilisation dorsale ou palmaire.

Il en est ainsi pour l'orthèse d'immobilisation décrite dans US-A-4 883 073 qui, soit droite, soit gauche, ne peut être utilisée qu'avec son éclisse principale en position dorsale, et du poignet de maintien, et non d'immobilisation, décrit dans US-A-5 267 943 qui, également droit ou gauche, ne peut être posé qu'avec ses baleines élastiques en position palmaire.

De même, le poignet, décrit dans US-A-5 513 657, n'assure qu'un maintien et non une immobilisation, en raison de sa forme, de sa position et du recours à des baleines flexibles, disposées dans des poches parallèles fermées, et a un usage unique pour une main droite ou gauche.

L'orthèse de maintien décrite dans US-A-5 014 689 comporte une enveloppe qui s'étend des articulations métacarpo-phalangiennes jusqu'en deçà du poignet, et qui est munie dans ses parties dorsales et palmaires de poches contenant des baleines flexibles. Cette orthèse est à usage unique, main droite ou main gauche et n'est pas réversible. De plus, la partie distale et palmaire de son enveloppe vient en avant de la ligne d'articulation S et gène le mouvement vers le bas des phalanges.

Pour satisfaire à la diversité des tailles, ces orthèses doivent en plus être réalisées en un grand nombre de modèles

Pour limiter ce nombre de modèles, entraînant des coûts élevés de fabrication et de gestion des stocks, ont été crées des modèles ambidextres, droite ou gauche, ou polyvalents, à savoir utilisables en position palmaire ou dorsale. Toutefois, cette augmentation des possibilités d'utilisation a nécessité des concessions importantes sur l'adaptation à l'asymétrie de la main, au détriment de l'efficacité de l'immobilisation du poignet ou de la liberté de mouvements des phalanges.

La présente invention a pour objet de fournir une orthèse universelle, couvrant, par un nombre de modèles très réduits, la totalité des applications et utilisations, tout en assurant une excellente immobilisation du poignet et en laissant une totale liberté de mouvements aux phalanges.

Cette orthèse est également du type dans lequel l'enveloppe présente au moins localement des nappes de velours à bouclettes aptes à coopérer avec des nappes de velours à crochets portées par des sangles transversales de maintien.

Dans l'orthèse selon l'invention, les poches, sont réalisées dans la partie centrale de l'enveloppe apte à venir sur le dos de la main ou sous la main, sont munies d'extrémités proximales, fermées et parallèles, et d'extrémités distales ouvertes, tandis que, de première part, les éclisses sont munies chacune d'une partie distale coudée apte à épouser le dos ou l'intérieur de la main, sont rigides, amovibles et retournables dans leur poche, et ont des longueurs différentes de manière que, lorsqu'elles sont engagées à fond dans leurs poches respectives, la droite S joignant leurs extrémités distales soit sensiblement parallèle à l'axe de répartition des articulations métacarpo-phalangiennes, que de deuxième part, l'enveloppe est prolongée au-delà de la partie ouverte des poches par un rabat de fermeture muni de moyens d'accrochage sur les poches et d'une charnière souple apte, par appui sur l'extrémité distale des éclisses, à se plier sensiblement parallèlement à l'axe de répartition des articulations métacarpo-phalangiennes, à l'aplomb duquel cette charnière est disposée, et que, de troisième part, les sangles de maintien sont indépendantes de l'enveloppe et comprennent une sangle distale passant entre pouce et index et deux sangles transversales, respectivement, intermédiaire et proximale.

Pour adapter l'orthèse à la main, droite ou gauche, et au type d'utilisation, palmaire ou dorsale, il suffit donc de modifier la position relative des éclisses dans les poches, de manière que lors du repliement du rabat, le bord de la charnière ait sensiblement la même inclinaison que celle de répartition des articulations métacarpo-phalangiennes.

Les éclisses sont retournées sur elles-mêmes lors du passage de la position dorsale à la position palmaire, ou inversement, de manière que leurs extrémités coudées suivent la cambrure du dos de la main ou de la paume de la main.

Dans une forme d'exécution de l'invention, les poches sont constituées par une nappe de velours à bouclettes fixée sur l'enveloppe souple, tandis que le rabat, également constitué par une nappe de velours à bouclettes dont les bouclettes sont extérieures, comporte, sur la face interne de son extrémité, une nappe de crochets apte à coopérer avec les bouclettes des poches.

Ces moyens de fermeture permettent d'assurer la liaison du rabat avec l'enveloppe, quelle que soit l'angulation de la charnière.

Avantageusement, l'enveloppe est composée, côté peau, d'une nappe en matériau anallergique et, côté extérieur, d'une nappe présentant des bouclettes extérieures sur toute sa surface et constituant moyens d'accrochage pour les extrémités, dotées de nappe à crochets, des trois sangles indépendantes, respectivement antérieure, intermédiaire et postérieure.

Ainsi, l'enveloppe est indépendante de ses sangles de fixation sur la main qui peuvent ainsi être positionnées en fonction de la morphologie de la main. Plus précisément, la sangle distale passant entre le pouce et l'index et contre la paume ou le dos de la main, contribue efficacement au positionnement longitudinal de l'orthèse par rapport aux articulations métacarpo-phalangiennes, mais aussi à l'immobilisation longitudinale de l'orthèse sur le poignet et cela en combinaison avec la sangle intermédiaire, qui peut être disposée à proximité immédiate du pouce pour assurer un calage parfait de l'orthèse.

La combinaison des éclisses de longueurs différentes, positionnables en fonction des besoins, de la structure générale de l'orthèse et des trois sangles indépendantes, permet, avec une plage limitée de pointures, d'adapter cette orthèse à toutes les applications, que la main soit droite ou gauche et que le maintien soit palmaire ou dorsal, en donnant ainsi à cette orthèse un caractère polyvalent, tout en assurant l'immobilisation du poignet et une totale liberté des phalanges.

D'autres caractéristiques et avantages ressortiront de la description qui suit en référence au dessin schématique annexé représentant une forme d'exécution de cette orthèse.
Figures 1 et 2 sont des vues respectivement en plan par dessous et en plan par dessus d'une main gauche,
Figures 3 et 4 sont des vues en plan par dessus de l'orthèse selon l'invention, lorsqu'elle est conformée, respectivement, pour une application palmaire contre la main de figure 1, et pour une application dorsale contre la main de figure 2,
Figures 5 et 6 sont des vues de côté montrant la position des éclisses dans le cas d'une application palmaire et dorsale,
Figure 7 est une vue en plan par dessus montrant les différents éléments composant l'orthèse,
Figure 8 est une vue en plan par dessus de l'enveloppe lorsqu'elle est à plat,
Figures 9 et 10 sont des vues en perspective montrant l'orthèse lorsqu'elle est mise en place et sanglée, respectivement dans le cadre d'une application dorsale et dans le cadre d'une application palmaire.

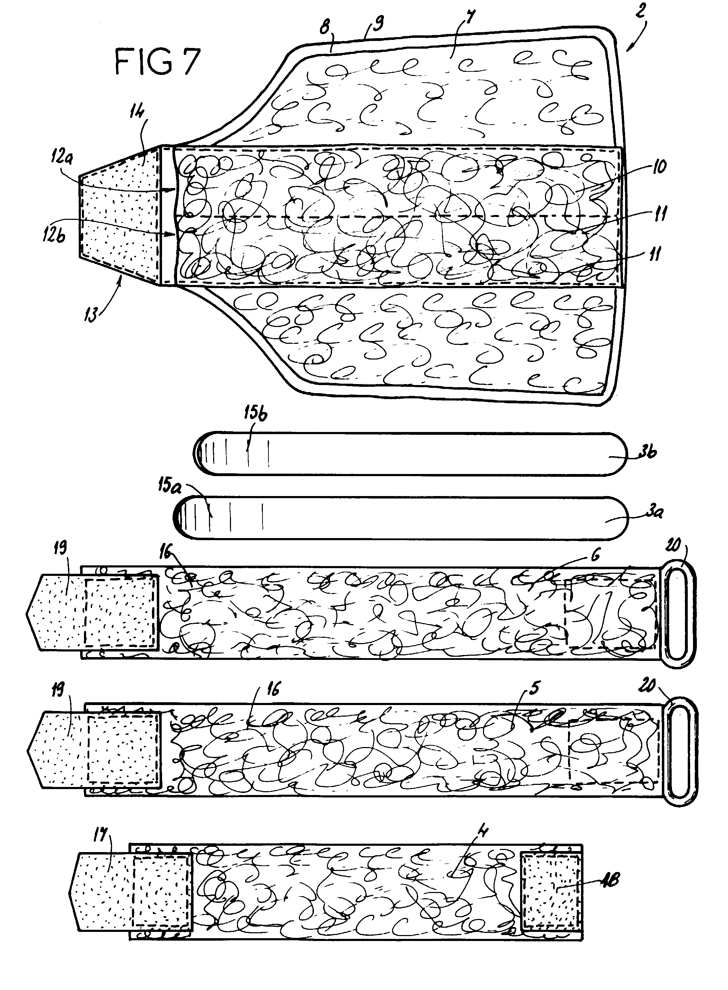

Comme montré à la figure 7, l'orthèse selon l'invention est composée d'une enveloppe souple 2, d'au moins deux éclisses rigides 3a, 3b et de trois sangles indépendantes, à savoir une sangle distale 4, une sangle intermédiaire 5 et une sangle proximale 6.

L'enveloppe souple 2 est composée, côté peau, d'une nappe en matériau anallergique, non représenté, et, côté extérieur, d'une nappe 7 présentant des bouclettes extérieures sur toute sa surface. Ces deux nappes sont liées l'une à l'autre par une couture périphérique 8 assurant également la fixation d'un galon 9. Une nappe 10, également eh velours à bouclettes avec les bouclettes tournées vers l'extérieur, est fixée par des coutures 11 sur la partie centrale de l'enveloppe 2, c'est-à-dire sur la partie apte à venir contre le dos de la main ou contre la face palmaire de celle-ci. Les coutures 11 délimitent deux poches longitudinales 12a, 12b, de même longueur et présentant des extrémités proximales parallèles, fermées par une couture transversale, et une extrémité distale ouverte et débouchant de la partie distale de l'orthèse.

L'enveloppe 2 a une longueur lui permettant de s'étendre longitudinalement depuis la ligne S jusqu'en deçà du poignet et les poches 12a s'étendent sur toute sa longueur. Elle est prolongée sur l'avant des poches par un rabat 13, dont la partie côté peau est réalisée en velours à bouclettes. Ce rabat comporte, sur sa face qui est extérieure lorsqu'il est dans le prolongement de l'enveloppe 2, une nappe 14 de crochets aptes à coopérer avec les bouclettes des poches.

Les deux éclisses rigides 3a et 3b sont réalisées en métal ou en matière synthétique. Elles ont des longueurs différentes, celle 3a étant plus longue que celle 3b. A proximité de son extrémité distale, chacune des éclisses est coudée pour former, avec sa partie distale 15a, 15b, un "V" ouvert s'adaptant à la main en fonction de l'orientation de sa concavité, comme montré figures 5 et 6.

Chacune des sangles indépendantes 4, 5, 6 comporte, au moins sur sa partie extérieure, une nappe de velours à bouclettes 16. La sangle distale 4 porte, à chacune de ses extrémités, des tronçons respectivement 17 et 18 de nappe de velours à crochets ou de crochets en matière synthétique, tandis que les sangles, respectivement, intermédiaire 5 et proximale 6, comportent, à une extrémité, un tronçon de nappe de velours à crochets 19 et, à l'autre extrémité, une boucle 20, améliorant le serrage.

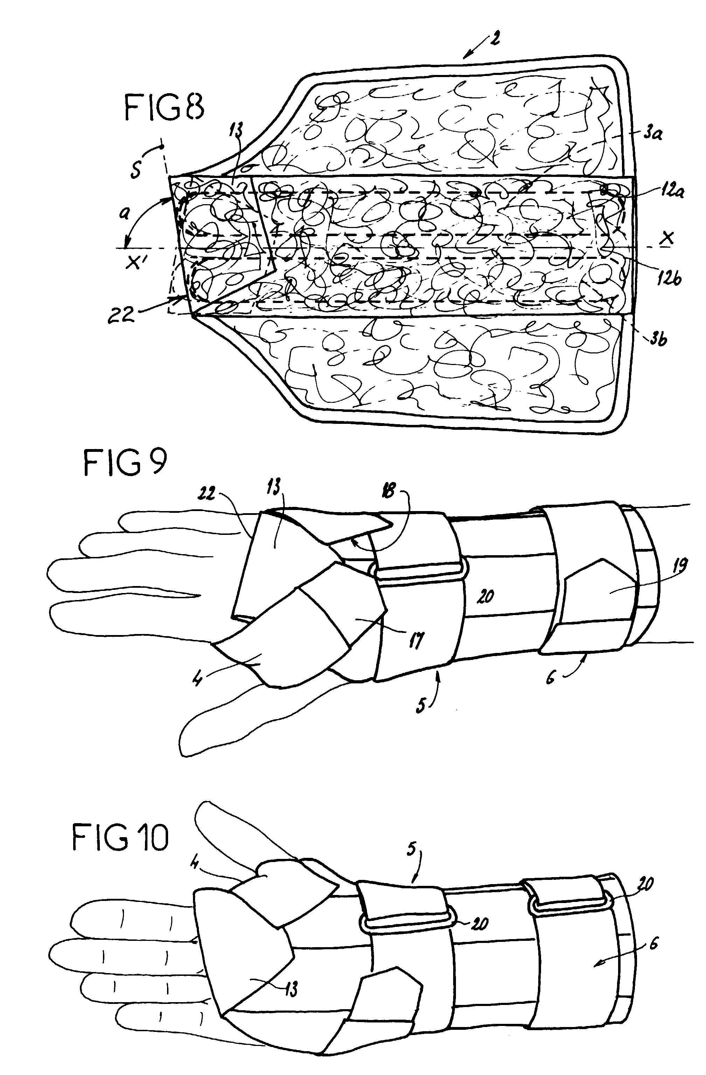

La figure 8 montre que, lorsque les éclisses 3a, 3b sont engagées à fond dans les poches 12a, 12b, et que le rabat 13 est rabattu sur l'enveloppe 2 de manière que sa charnière 22 reste en contact avec l'extrémité de chacune des éclisses, cette charnière n'est pas perpendiculaire à l'axe longitudinal x'-x de l'orthèse, mais est inclinée par rapport à cet axe d'un angle a, différent de 90°. Cette même figure montre, qu'en inversant la position des éclisses 3a, 3b dans les poches 12a, 12b, l'inclinaison peut être inversée symétriquement. Cela permet donc, comme montré aux figures 3 et 4, d'adapter l'inclinaison de l'extrémité de l'orthèse, de manière que cette dernière soit voisine de l'inclinaison des articulations métacarpo-phalangiennes, représentées par les traits mixtes S aux figures 1 et 2.

Les figures 5 et 6 montrent qu'en retournant les éclisses 3a, 3b dans les poches 12a, 12b, la partie distale coudée 15a, 15b des éclisses permet d'adapter parfaitement leur forme au profil de la main, quelle que soit l'application, palmaire ou dorsale.

Il ressort de ce qui précède que l'orthèse selon l'invention peut aussi bien être utilisée en position dorsale qu'en position palmaire, sur une main droite ou sur une main gauche, en ayant une extrémité distale qui est inclinée sensiblement parallèlement aux articulations métacarpo-phalangiennes et qui, de ce fait, ne gêne en rien le mouvement de ces articulations.

Un autre avantage de cette disposition est, qu'avec un nombre réduit de modèles, par exemple trois, respectivement petit, moyen et grand, il est possible de couvrir toutes les applications, ce qui réduit les coûts de fabrication, les coûts de gestion des stocks, et le prix de revient de l'orthèse.

La mise en place de l'orthèse s'effectue en la positionnant longitudinalement de manière que la charnière 22 soit sensiblement à l'aplomb au niveau de l'articulation métacarpo-phalangienne, puis en l'immobilisant provisoirement dans cette position au moyen de la sangle distale 4. Dans le cas d'une application palmaire, cette sangle passe sous la paume de la main, comme montré à la figure 9, tandis que dans une application dorsale, elle passe sur le dos de la main (figure 10). La fixation temporaire est assurée en appliquant les extrémités à crochets 17 et 18 de la sangle 4 sur les bouclettes constituant le dos de l'enveloppe 2, le dos des poches 12a, 12b et le dos du rabat 13.

Il est alors procédé à la mise en place de la sangle intermédiaire 5, qui est disposée très près du pouce, puis à la mise en place de la sangle proximale 6. Pour chacune de ces deux sangles, le verrouillage est assuré par accrochage des tronçons de velours à crochets 19 dans les bouclettes du dos de la sangle.

A ce stade de la mise en place, et si besoin est, la sangle distale 4 est ajustée en position.

A la fin de la mise en place, et comme montré aux figures 9 et 10, l'orthèse est parfaitement positionnée longitudinalement et est calée en translation longitudinale par les sangles, respectivement distale 4 et intermédiaire 5, disposées de part et d'autre du pouce.

Le recours aux sangles indépendantes 4 à 6 permet d'assurer un bon positionnement de l'orthèse, quel que soit son mode d'utilisation palmaire ou dorsale, quelle que soit la forme générale de la main et du poignet et contribue donc à améliorer l'immobilisation du poignet, sans gêne anormale.

Grâce à ce maintien, il n'est pas indispensable que l'enveloppe entoure tout le poignet, ce qui améliore le confort.

Les éclisses qui, dans la forme d'exécution représentée, sont au nombre de deux, peuvent également être au nombre de trois et coopérer avec trois poches, parallèles et juxtaposées, ménagées sur l'enveloppe.

## Revendications

1. Orthèse de poignet ambidextre, dorsale ou palmaire, composée d'une enveloppe souple (2) s'étendant longitudinalement des articulations métacarpo-phalangiennes jusqu'en deçà du poignet et comportant plusieurs poches longitudinales, parallèles et juxtaposées (12a, 12b) pour des éclisses (3a, 3b), cette enveloppe présentant au moins localement des nappes de velours à bouclettes aptes à coopérer avec des nappes de velours à crochets portées par des sangles transversales de maintien, **caractérisée en ce que** les poches (12a, 12b), sont réalisées dans la partie centrale de l'enveloppe (2), apte à venir sur le dos de la main ou sous la main, sont munies d'extrémités proximales, fermées et parallèles, et d'extrémités distales ouvertes, tandis que, de première part, les éclisses (3a, 3b) sont munies chacune d'une partie distale coudée (15a, 15b) apte à épouser le dos ou l'intérieur de la main, sont rigides, amovibles et retournables dans leur poche, et ont des longueurs différentes de manière que, lorsqu'elles sont engagées à fond dans leurs poches respectives, la droite S joignant leurs extrémités distales soit sensiblement parallèle à l'axe de répartition des articulations métacarpo-phalangiennes, que de deuxième part, l'enveloppe (2) est prolongée au-delà de la partie ouverte des poches (12a, 12b) par un rabat de fermeture (13) muni de moyens d'accrochage sur les poches et d'une charnière souple (22) apte, par appui sur l'extrémité distale des éclisses (3a, 3b), à se plier sensiblement parallèlement à l'axe (S) de répartition des articulations métacarpo-phalangiennes, à l'aplomb duquel cette charnière est disposée, et que, de troisième part, les sangles de maintien sont indépendantes de l'enveloppe et comprennent une sangle distale (4) coopérant avec le pouce et deux sangles transversales, respectivement, intermédiaire (5) et proximale (6).

2. Orthèse universelle selon la revendication 1, **caractérisée en ce que** les poches (12a, 12b) sont constituées par une nappe (10) de velours à bouclettes fixée (11) sur l'enveloppe souple (2), tandis que le rabat (13), également constitué par une nappe de velours à bouclettes, dont les bouclettes sont extérieures, comporte, sur la face interne de son extrémité, une nappe (14) de crochets aptes à coopérer avec les bouclettes des poches.

3. Orthèse selon l'ensemble des revendications 1 et 2, **caractérisée en ce que** l'enveloppe (2) est composée, côté peau, d'une nappe (7) en matériau anallergique et, côté extérieur, d'une nappe présentant des bouclettes extérieures sur toute sa surface et constituant moyens d'accrochage pour les extrémités, dotées de nappe à crochets (17, 19), des sangles (4 à 6).
